# EUROPEAN PATENT SPECIFICATION

(11) **EP 0 292 630 B1**
(45) Date of publication and mention of the grant of the patent: **08.03.1995**
(21) Application number: 87710015.6
(22) Date of filing: 06.10.1987
(51) Int. Cl.: A61N 5/10

(54) **Radiation therapy delivery systems**
Zuführsysteme für die Strahlentherapie
Systèmes de délivrance en radiothérapie

(30) Priority: 30.03.1987 US 31885
(43) Date of publication of application: 30.11.1988
(73) Proprietor: Horowitz, Bruce S., Dr., Ft. Lauderdale, Fl. 33326 (US)
(72) Inventor: Horowitz, Bruce S., Dr., Ft. Lauderdale, Fl. 33326 (US)
(74) Representative: Allam, Peter Clerk

(56) References cited:
- EP-A- 0 152 124
- EP-A- 0 158 630
- EP-A- 0 185 939
- WO-A-86/04248
- FR-A- 2 240 746
- GB-A- 2 138 298
- US-A- 3 750 653

## Description

This invention relates to delivery systems for temporary radiation therapy.

Interstitial radiation therapy has been performed since the beginning of the 20th Century. Radium was developed by Madam Curie and Alexander Graham Bell proposed the use of radium in tumors. Subsequently, metal needles were developed in which a radium isotope was encapsulated for insertion in close proximity or into tumors. Where the tumor was deep seated, an operation was necessary to provide access to the tumor. Such therapy had serious problems and disadvantages. The high energy of the radium isotope required a great deal of shielding and subjected the personnel to harmful exposure. In addition, the needles tended to break as they aged resulting in the release of the radioactive contents. Since the radium isotopes had a half-life of about 1600 years, they produced an extreme contamination hazard.

Thus, efforts have been made to develop more effective brachytherapy which is safer and more convenient to use. This has resulted in the development of radioactive materials that have lower energies and thus require less shielding and have shorter half-lives to reduce the risk of contamination. Thus, permanent seeds of encapsulated radon-222 having an energy level of encapsulated gold-198 having an energy level of 0.42 MEV and a half-life of 2.7 days have been used. More recently small seeds of iridium-192 having an energy level of 0.30 MEV and a half-life 74.2 days and iodine-125 having an energy level of 0.028 MEV and a half-life of 60 days have been developed. Such seeds are shown, for example, in U.S.-A-3,351,049 and US-A-4,323,055.

Such iridium and iodine seeds are on the order of 4.5 mm in length and 0.8 mm in diameter and are implanted in the tumor or placed in the surface of the tumor. Both of these sources have lower energies than radium that allow for simpler shielding and less radiation exposure to personnel. With seeds of iodine encapsulated in a material such as titanium, shielding is provided by the surrounding tissue and the seeds can be left in the patient permanently without the need for major precautions.

A further development in brachytherapy has been the development of techniques for implanting the newer developed radioactive seeds and the development of techniques for handling the seeds. Two techniques, in general, have been developed to allow further reduction in personnel exposure during the implanting process. The two techniques involved are for permanent and temporary implants. Both utilize after loading techniques where guides in the form of catheters are placed through the tumor mass or on the tumor surface.

Permanent implants usually use sources with a very short half life of a few days or low energy low activity, both resulting in very low exposure to bystanders so the patient can be discharged without fear. Since loose radioactive seeds are utilized, this procedure can only be used by volume implants in an effort to firmly hold the seeds in place. Problems arise if an implant is needed in a volume that will not be able to sustain the seeds in a rigid geometrical position, such as a breast tumor where the breast tissue is flexible. Likewise, the tumor volume only represents a thin layer of tumor cell. Examples include tumors that grow over the surface but invade only minimally the skin or a flat plexus of vessels containing tumor such as the tumor barring lymphatics of the mediastium or the tumor bed when a tumor is removed off of a surface which still harbors malignant cells. This type of implant usually requires a supporting carrier for the radioactive seeds, which must be removed within a specified time, necessitating a temporary implant. Thus, in this technique nylon plastic catheters are sutured on, or pierced through, the tumor volume. The catheters are then loaded by inserting a second nylon catheter of a smaller diameter containing the radioactive seeds. After the desired radiation dose is delivered, the two catheters are withdrawn.

The temporary catheter placement for treatment of tumor beds has an ever increasing role in cancer managements as mutilating surgical procedures decrease and post operative irradiation is utilized to treat the scattered cancer cell. Thus the use of placing temporary iridium implants as described above has found a significant role in the treatment of lung tumors. Tumors frequently have to be sharply dissected off of the chest wall, diaphragm, or the central vital organs of the chest. Colon and rectal tumors frequently have to be dissected off of the pelvic bony wall. Although this technique does work, there are inherent problems that this invention solves while other techniques attempting to solve the problem do not address all the to be described difficulties and do not solve them.

As described above the technique for temporary planar implants involves the suturing of nylon catheters to the tumor bed after the removal of the cancer while the patient is under general anesthesia. The method is well described Hilaris et al, "Value of Perioperative Brachytherapy in the Management of Non-Oat Cell Carcinoma of the Lungs", Int. J. Radiation Oncology Biol. Phys.; Vol. 9, pp. 1161-1166, (1983). Each catheter should be sutured in three to four places. This is a time consuming procedure which uses expensive operating time as well as increasing the patient's risks. This problem is compounded if the implant needs to be performed in a tight area or awkward position. The catheters cannot be sutured at all when removal of the tumor is so complete as not to leave tissue to suture the catheters to. An example of this would include a pelvic tumor removed leaving only pelvic bone riddles with cancer cells. Since these catheters are anchored in only at a few points along their path, yet need to follow an irregular surface, the catheters tend to lose the parallel spacing between them, resulting in areas where the catheters become bunched together, and a resultant relative hot spot may occur causing radiation overdose with necrosis, while leaving an area of underdosage where tumor recurrence is a possibility. An overdose over a critical area such as the spinal cord can leave a patient crippled for life, while tumor recurrence will cause the patient's death.

Another technique described by Martinez, et al, "Sterilization of 125₁ Seeds Encased in Vicryl Sutures for Permanent Interstitial Implementation", Int. J. Radiation Oncology Biol. Phys.; Vol. 5, pp. 411-413 (1979) utilizes Iodine-125 seeds spaced within a braided absorbable polymer surgical suture (Vicryl). Although this technique allows for a permanent planar implant in areas described above, this technique still retains many of the above problems of time consumption of placing individual sutures, including inability to implant denuded areas, and ability to maintain exact parallelism between the ribbons. Further, the seeds move within the sutures during attachment to the tumors thereby eliminating longitudinal spacing of the seeds.

Another technique by Marchese, et al, "A Versatile Permanent Planar Implant Technique Utilizing Iodine-125 Seeds Imbedded in Gelfoam", Int. J. Radiation Oncology Biol. Phys.; Vol. 10, pp. 747-751 (1984) again utilize absorbable materials and Iodine-125 seeds to perform a permanent planar radioactive implant. This technique utilizes commercially available absorbable polymer mesh and putty material. At the time of the surgical debulking of tumor, the mesh is cut to conform to the tumor bed, the putty is flattened into pancake fashion, and the deeds are placed into the putty, at a predetermined spacing then placed on the mesh which is sutured over the tumor bearing surface. Although this technique solved the problem of planar implants over denuded areas, and reduces actual suturing time, new problems are created. First, since the pancake style implant can only be manufactured after the tumor is removed and the shape of the tumor bed is known, and since it has to be manufactured under sterile conditions, the end result is a greatly extended operating time while the pancake is being prepared. Another problem is there is no quality assurance of the thickness of the pancake holding the radioactive seeds, and thus in thin areas the material will be absorbed quickly resulting in premature release of the seeds, under dose in that area, and recurrence of tumor.

It is also noted in the techniques of Hilaris and Martinez above that the seeds are low dose rate, and would not be applicable in a rapidly proliferating tumor system.

Delivery systems have been developed using applicators designed to place radioactive sources either on the surface, interspread throughout the tissues (interstitial) and intracavitary in the tissue surrounded by tumor (intracavitary). Interstitial placement of seed requires the placement of hollow metal needles inserted through the normal tissues and into the tumor. After inserting the needles in the tumor, the seeds are thereafter inserted into the needles, while the needles are being retracted to deposit the seeds in the tumor. Such devices are shown in US-A-4,402,308. The most commonly used instruments are the Henschke and Mick devices. The spacing of the needles is determined by a nomograph developed by Drs. H.M. Kuam and L.L. Anderson of the Department of Medical Physics at Memorial Sloan-Kettering Cancer Center, New York. The use of such devices has distinct disadvantages and problems. The overall length of such devices is over 20 cm and they have significant weight making them difficult to manipulate. Since the implant is performed through an open surgical wound, the needles can only be placed straight in a straight line or at an angle dictated by the relationship of the incision to the tumor For example, the prostate is directly below the pubic bone with the incision being located cephalad. Since the prostate tends to rise behind the bladder, the preferred direction of the implant should be from a caudal approach, but this is not achievable using the available devices. Another disadvantage of the above technique is that the seeds are deposited in a track made by the needle. When the needle is withdrawn, there is a tendency for the seeds to migrate in the needle track resulting in a poor distribution of the seeds. Because the energy levels are low (an exposure constant of 0.0184 for iodine-125 vs. 0.825²Rm²ci¹h¹ for radium), the distribution between centers of adjacent seeds should be on the order of 1 cm. Poor distribution of seeds can result in undesirable concentrations of seeds resulting in either an over dosage or underdosage of radiation.

The seed is small because it needs to fit in small bore needles which minimally change or damage tissue. The seed has a high seed surface dose and is difficult to label. In addition, the technique of implantation of individual seeds is time consuming.

In another technique for treating tumors, seeds are initially placed by hand in a woven or braided absorbable carrier such as a braided suture. The carrier with the seeds laced therein is then secured in place to form a suitable implant. This technique is time consuming and may necessitate handling of the suture as well as having the same problems as to position and retention as the catheters. In order to minimize the radiation to personnel during handling and shipping, the suture with the seeds placed therein is shielded by placing it in a curved metallic tube, see EP-A-0 064 860.

In another technique that has been developed for treatment of tumors, plastic catheters are sutured on or in the tumor area and the seeds are placed in the catheters by insertion of a nylon tube carrying the seeds. After the desired treatment period, the nylon tubes are removed. The catheters are difficult to place so as to provide the proper dose distribution. It is also difficult to accurately space the catheters in parallel array over irregular surfaces and to anchor the catheters to the tissue. Irregular spacing can result in radiation overdose or underdose. Where the ends of the catheters are brought to the surface of the skin and sutured in place, there is an incipient source of contamination.

The intracavitary placement of radioactive seeds is a method that pertains to this invention. Intracavitary brachytherapy provides the ability to place radioactive material within and around a tumor to give the advantage of a high local tumor dose with normal tissue sparring as compared with an external beam apparatus alone. Intracavitary brachytherapy of gynecological tumors, due to the accessibility of the vaginal and uterine cavities, allow easy insertion of radioactive sources. Today intracavitary brachytherapy has considerably improved local control and survival when using radiotherapy. It is known at this time that when cervical carcinoma is locally extensive the combination of brachytherapy and external beam has doubled survival over radical surgery alone. Today the Fletcher-Suit intracavitary applicator is the most commonly used gynecological applicator. Its development incorporates features found most important for local tumor control and minimized complications. These factors include fixation of the radioactive source geometry in relation to the normal pelvic structures, and separating the normal structures adjacent to the applicator from the sources. The system consists of a tandem used to hold radioactive sources in the uterine cavity, thus allowing a radioactive material to be located close to the tumor's center. Two hollow cylinders are placed in the vaginal fornices that hold radioactaive seeds within the center of each cylinder. These cylinders are stainless steel and are actually modifications of the hollowed corks used in the Manchester system. The cylinders are attached to handles, which allow stabilization of the tandem with respect to the pelvis. It is well recognized now that the tandem and the cylinders improve the dose distribution by spacing the adjacent normal tissues from the radioactive source surface. Due to the exponential drop in dose rate as a function of distance, one is able to decrease local necrosis due to high surface doses of adjacent tissue as compared to radiation given at depth. This has been elaborated on by Rottman, M. in a publication titled "Intercavitary Applicator in Relation to Complications of Pelvic Irradiation", published in the International Journal of Radiology, Oncology, and Physics, 4:951-956, 1978.

One embodiment of the invention describes how to allow radiation treatment to be delivered by intracavitary means to what was previously considered to be inaccessible site.

An example of such a site includes the brain. At the time of craniotomy a cavity is formed when the tumor is removed from the brain. The craniotomy carries a significant morbidity, and possible mortality due to anesthetic risks as well as infectious and hemorrhagic complications. Thus, eliminating the necessity of a second surgical procedure to remove a radioactive source and eliminating a second incision that can act as a portal for bacteria would increase the applicability of using intracavitary brachytherapy. A permanent implant would allow for immediate closure of the surgical wound and remove the necessity of a second surgical procedure.

Prior efforts to treat tumors in the brain have been by insertion of catheters and radioactive sources into the tumor or by removing the tumor by surgical techniques. In each instance, it has been difficult to insure whether proper treatment has been made and that the entire tumor has been properly treated.

In general, none of the prior art systems allow for the adequate placement of radioactive seeds in the configuration, shape or size of the tumour treated.

Therefore, it would be desirable to be able to provide a delivery system for temporary radiation therapy which is capable of placing and holding the seeds in the desired and accurate position, with respect to the configuration of the tumor, to provide the desired radiation dose in a controlled manner. Such a delivery system should be safe and easy to use and cause a minimum of trauma to tissue.

With respect to intracavitational radiation therapy, it would be desirable to provide a delivery system which will deliver proper radiation in instances where the tumor has been surgically removed; which system will continue to treat the surface area of the brain without necrosis of normal tissue and which can be permanently left in the brain.

In accordance with the present invention there is provided a delivery system for radiation therapy which, as in the system described by Martinez et al, supra, comprises an absorbable template made of a material absorbable in living tissue and radioactive seeds supported by the template in a predetermined array. The delivery system of the present invention is characterized in that each said radioactive seed is individually pre-encapsulated by the template such that said radioactive seeds of said array are in fixed stationary positions relative to each other upon implantation of said template in a patient.

In accordance with one embodiment of the invention, the delivery system comprises a template in the form of a conformable sheet of material absorbable in living tissue which has a plurality of individually pre-encapsulated radioactive seeds dispersed therein in a predetermined and fixed array. In one form, the sheet comprises a continuous flexible film having the radioactive seeds individually pre-encapsulated therein and which sheet may be attached to the surface of a tumor.

In accordance with an embodiment of the invention particularly relating to interstitial radiation therapy, the delivery system comprises a template in the form of a substantially non-deflecting member absorbable in living tissue. The member has a length that greatly exceeds its width or diameter. The non-deflecting member has a plurality of radioactive seeds individually pre-encapsulated therein in a predetermined and fixed array. In one form, the non-deflecting member comprises an elongated implant in the form of a needle for insertion in a tumor.

An embodiment of the invention suitable for intracavitational brachytherapy comprises a body of material which is absorbable in animal tissue and a plurality of radioactive seeds within the body and individually pre-encapsulated in the body. In one form, the body is hollow with an exterior wall defining separate central chamber such that the hollow body exterior wall structure will accommodate growth of body tissue such as brain tissue. In another form, the body comprises a solid body having the seeds held in the center of the body that can be inserted in the cavity to be treated. In another form, a flat sheet is attached tangentially to the hollow exterior wall to facilitate placement over the brain.

These and other embodiments of the delivery system of the present invention will now be described in detail, by way of example, and with reference to the accompanying drawings, in which:
Fig. 1 is a perspective view of a first embodiment of the delivery system in the form of a tumor conformable sheet;
Fig. 2 is an enlarged sectional view taken along line 2-2 in Fig. 1;
Fig. 3 is an enlarged sectional view of a typical seed;
Fig. 4 is a perspective view of another embodiment of a conformable sheet delivery system;
Fig. 5 is a plan view of another embodiment of a conformable sheet delivery system;
Fig. 6 is a cross-sectional view taken along line 6-6 in Fig. 5;
Fig. 7 is an enlarged side view of an interstitial delivery system in the form of a needle;
Fig. 8 is a greatly enlarged fragmentary sectional view on an enlarged scale of the needle shown in Fig. 7;
Fig. 9 is an enlarged sectional view of a typical seed;
Fig. 10 is a greatly enlarged perspective view of a part of the needle shown in Figs. 11 and 12;
Fig. 11 is an enlarged part sectional view of a portion of the needle shown in Fig. 15;
Fig. 12 is an enlarged view of another embodiment of the needle;
Fig. 13 is a diagrammatic view showing the manner of use of the needles;
Fig. 14 is an enlarged side view of a further embodiment of the needle;
Fig. 15 is an elevational view of an embodiment of the invention in the form of an intracavitational brachytherapy device;
Fig. 16 is a partial sectional view of the device shown in Fig. 15;
Fig. 17 is a full cross-sectional view of the device;
Fig. 18 is a view showing the device utilized in a cavity from which a tumor has been removed;
Fig. 19 is an elevational view of a further modified form of the intracavitational device;
Fig. 20 is a sectional view taken along line 20-20 in Fig. 19;
Fig. 21 is a plan view of the device shown in Fig. 19;
Fig. 22 is a longitudinal sectional view through a further modified form of the intracavitational device; and
Fig. 23 is a perspective view of the device shown in Fig. 22.

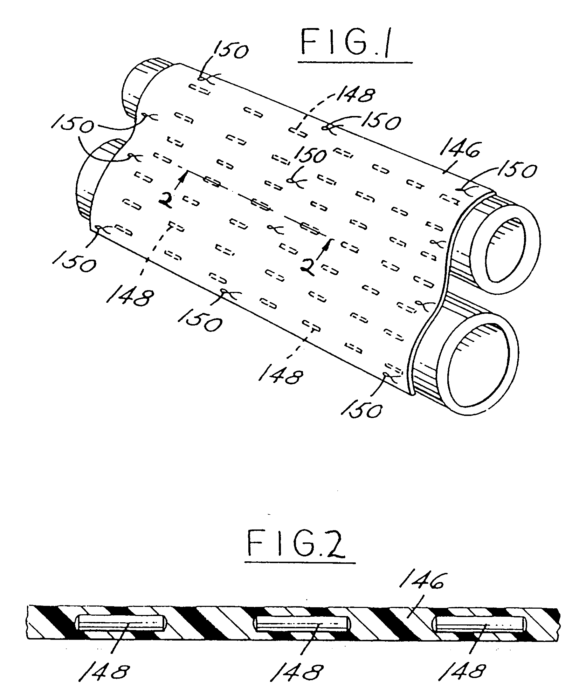

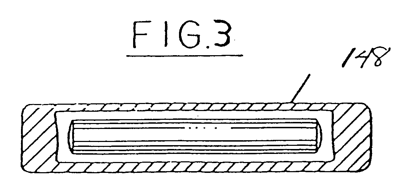

A delivery system with templates in the form of a conformable sheet material are shown in Figs. 1-6. The sheet material is absorbable in living tissue and is provided in the form of a flexible film for placing on and around tumors. The sheet may be attached adjacent a surface harboring a tumor. Radioactive seeds are positioned in a fixed and predetermined array or matrix in the sheet in the desired spaced relationships.

The seeds can be of various types having low energy and low half-life such as iodine seeds, known as I-125 seeds, consisting of a welded titanium capsule containing iodine 125 absorbed on a silver rod as shown in Fig. 2. Iridium seeds, designated Ir-192 seeds, can also be used.

The sheet may be made of any of the natural or synthetic absorbable materials such as suitable synthetic polyester amides derived from glycolic or lactic acids such as the polymers and copolymers of glycolide and lactide, and polydioxanone. Such polymeric materials are more fully described in US-A-3565869, US-A-3636956, US-A-4052988 and EP-A-30822. Specific examples of such polymers are sold by ETHICON, Inc., Somerville, New Jersey, under the trade marks "VICRYL" in "PDS".

The absorbable material should preferably maintain its integrity for from 1 to 14 days. Preferably the material should be absorbed in living tissue in a period of time from about 70 to 120 days. As little absorbable material as possible should be used. The sheet may be a cast sheet, extruded sheet, woven sheet, non-woven sheet of the like.

The radioactive seeds may be dispersed in the sheet in any desired pattern. The seeds are individually pre-encapsulated in the sheet; that is, they are each totally surrounded by absorbable material so that the sheet is safer and easier to handle.

The sheets of this embodiment of the present invention may be used in accordance with the following technique:
1. The tumor is exposed by a proper surgical technique.
2. The configuration of the tumor is determined.
3. The number and spacing of radioactive sources may be determined by the aforementioned nomograph technique developed by Drs. Kuam and Anderson. This calculation involves utilizing the average dimension and energy of the seeds as variables.
4. A suitable sheet having the desired spacing of seeds is cut to the appropriate size and placed on or around the tumor and preferably sutured or stapled in place.
5. After the sheet is in place, the surgical incision is closed.
6. Dosimetry is monitored using stereo shift orthogonal radiographs and the appropriate computer program.

In the form shown in Figs. 1-3, the delivery system is intended for use on the surface of a tumor or on a tumor bed and comprises a flexible continuous film 146 that can be con formed to the surface of the tumor or tumor bed and attached thereto as by suturing 150. The film is made of an absorbable material and a plurality of seeds 148 are positioned and individually pre-encapsulated in the film in a predetermined array of spaced seeds. In a typical example, the thickness of the film is 1.0 mm.

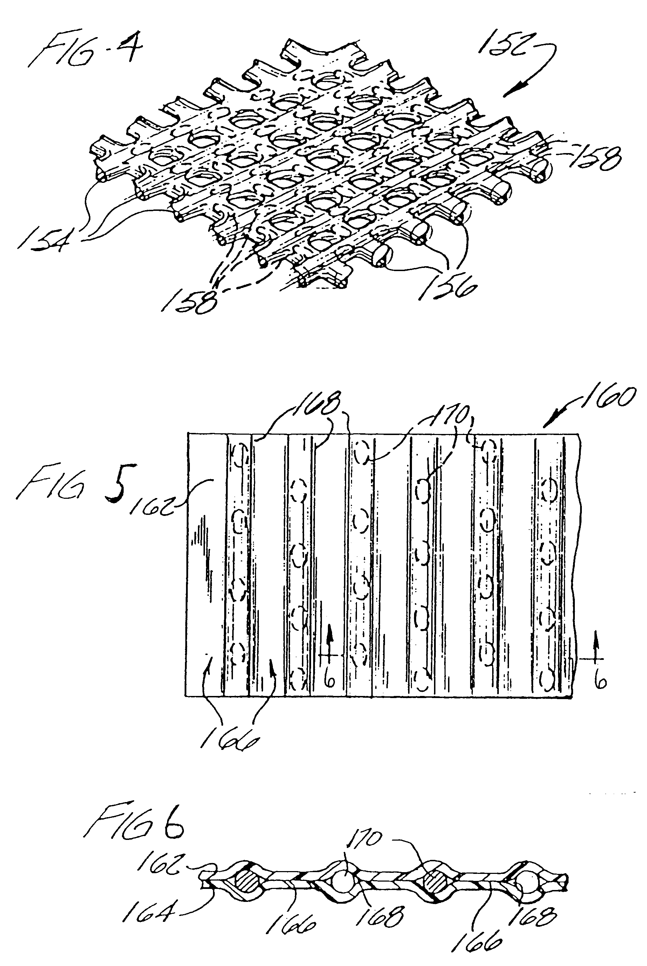

In the embodiment depicted in Fig. 4, the conformable sheet delivery system comprises a discontinuous film 152. The film comprises intersecting longitudinal strands 154 and transverse strands 156 which define perforations. In this embodiment radioactive seeds 158 are embedded in the longitudinal strands and the transverse strands at the desired spacing. A suitable spacing is 1 cm between the centers of adjacent seeds in each direction. The sheet may be attached to the tumor by suturing or stapling, as desired.

Referring to Figs. 5 and 6, there is shown a laminate 160 of two flexible films 162 and 164 of absorbable polymeric material. The films are secured together at intermittently spaced parallel rows 166. In the areas between the secured rows is a line 168 of radioactive seeds 170. Each line comprises a plurality of longitudinally aligned radioactive seeds. The film is also secured together between adjacent radioactive sources in a line.

The advantages of the improved con formable sheet delivery system are:
1. The sheets may be appropriately configured to the size and shape of the tumor.
2. Fixed linear positioning of seeds decreases or eliminates "hot" and "cold" spots.
3. As each seed is individually pre-encapsulated in the absorbable polymer, the normal tissue is spaced away from the seed surface by the thickness of the body of polymer to decrease necrosis from a high local dose.
4. Improved handling and labeling of the seeds will reduce exposure and error.
5. Increased speed of implant resulting in reduced surgical time and personnel exposure.
6. Ease of attachment and localized treatment in critical areas.

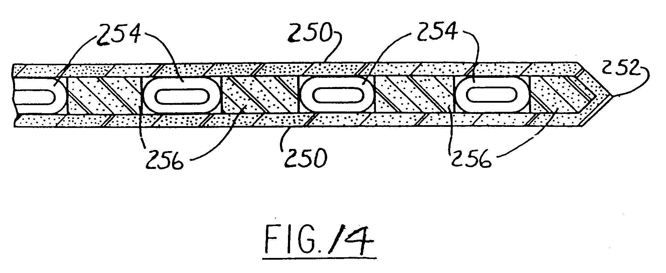

Figs. 7-14 show embodiments of the present invention relating to an improved delivery system for interstitial radiation therapy. A template in the form of a substantially non-deflecting elongated member made of material that is absorbable in living tissue is provided preferably in the form of a needle or thin pointed cylinder for insertion in tumors. A plurality of radioactive seeds are individually pre-encapsulated and positioned in a predetermined, fixed, spaced array in the body in the desired spaced relationship.

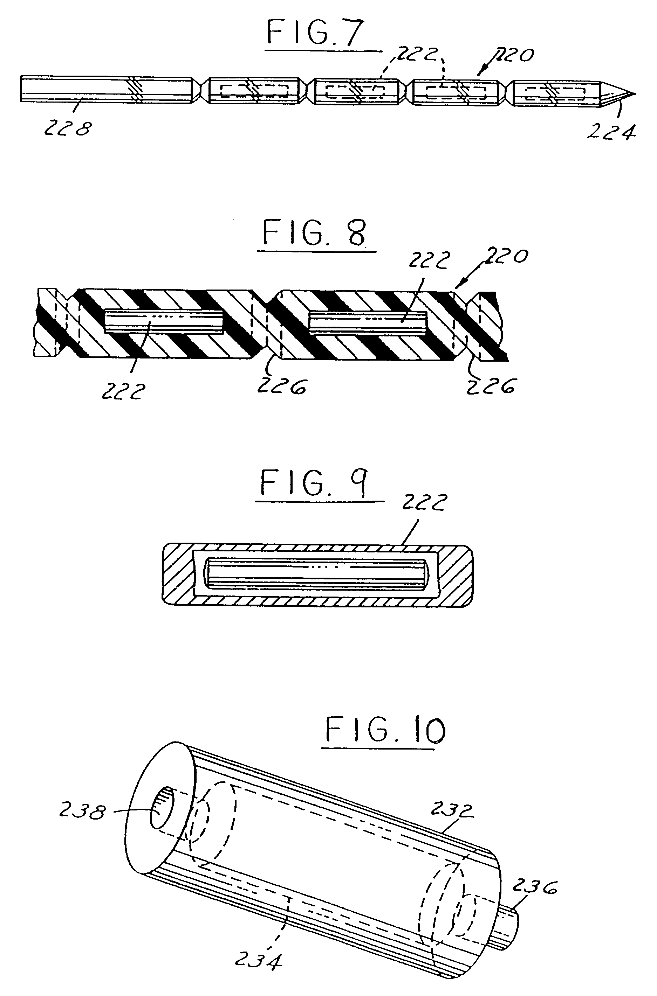

The seeds can be of various types having low energy and low half-life such as iodine seeds, known as I-125 seeds, consisting of a welded titanium capsule containing iodine 125 absorbed on a silver rod as shown in Fig. 9. Iridium seeds, designated Ir-192 seeds can also be used.

The non-deflecting elongated member may be made of any natural or synthetic absorbable material. Examples of natural absorbable materials are the polyester amides from glycolic or lactic acids such as the polymers and copolymers of glycolide and lactide, and polydioxanone.

The absorbable material should preferably maintain its integrity from 1 to 14 days. Preferably the material should be absorbed in living tissue in a period of time of from 70 to 120 days. It is preferred that as little absorbable material as possible be used in the delivery system of the present invention.

In the form shown in Figs. 7-9, the non-deflecting members comprise a needle 220 formed by an elongated plastic body in which the seeds 222 are encapsulated in an axially aligned and spaced relationships. The needle has a tapered end 224 and a surface of annular notches 226 are provided along the exterior surface in longitudinally spaced relation in the spaces between seeds so that the needle can be broken or cut to provide the proper length dependent on the size of the tumor. Alternatively, the needle can be marked by indicia rather than notched. In a typical case, the diameter of the needle is 1.06 mm.

The needles can be used in accordance with the following technique:
1. The tumor is exposed by a proper surgical technique. Alternatively, the tumor may be located by diagnostic methods using biplanar fluoroscopy, ultrasound or computerized tomography.
2. The size and shape of the tumor is determined.
3. The number of radioactive sources and spacing between the needles may be determined by the aforementioned nomograph technique developed by Drs. Kuam and Anderson. This calculation involves utilizing the average dimension and energy of the seeds as variables.
4. Each needle is inserted using one finger behind the tumor. When the end of the needle is felt, bluntly, the proper depth has been reached.
5. Portions of the needles extending beyond the tumor are removed by breaking or cutting between or beyond the seeds.
6. After all the needles are in place, the surgical incision is closed, of the tumor has been exposed by surgical technique.
7. Dosimetry is monitored using stereo shift orthogonal radiographs and the appropriate computer program.

In the form shown in Figs. 10-12 the needle 230 is in the form of a member comprising a plurality of segments 232 of absorbable material, each of which encapsulates a seed 234 and the segments 232 are physically interconnected to form the needle. Each segment 232 is formed with an integral projection 236 at one end and a complementary recess 238 in the other end for engagement with the projections 236 of an adjacent segment 232. An endmost tapered segment 240 is provided to facilitate insertion of the tumor. The needle 230 can be constructed by the physician or technician thereby creating a needle of a proper length. Each segment can be color coded for different strengths of radiation, for a spacer segment (i.e., segments without a radiation source) or otherwise.

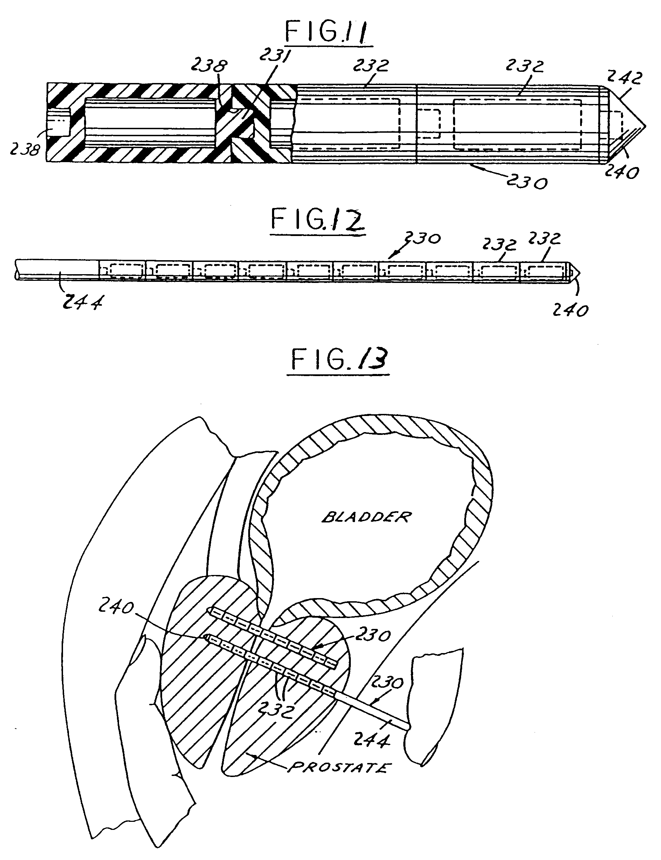

In each of the forms of needle heretofore described, a portion of the needle may be provided without seeds to form a handle 228 or 244 (as shown in Figs. 7 and 12, respectively) for handling and insertion, which portion can thereafter be removed form the remainder of the needle which is left in the tumor.

Fig. 14 shows another embodiment of the interstitial delivery system of the present invention. In this embodiment, a hollow tube 250 of absorbable material is used. One end 252 of the tube is beveled or tapered to form an end for insertion of the tube in a tumor. Radioactive seeds 254 are spaced in the tube along its length. The seeds are held in place in the tube by spacers 256 of absorbable material between each seed as depicted in Fig. 14.

It is preferred that the interstitial delivery system be inserted directly into the tumor being treated by merely determining the desired location for the radioactive seeds and then inserting the substantially non-deflecting elongated member into the tumor in a pattern which will provide the desired location for the seeds. However, if desired, a suitable hollow metal needle or trocar may be inserted in the tumor to facilitate placement of the non-deflecting elongated member.

Though it is contemplated that the interstitial delivery system may be inserted in tumors either with or without the assistance of some insertion device, such as a hollow needle, interstitial delivery system in accordance with the present invention should be substantially non-deflecting. By "non-deflecting" it is meant that the delivery system has sufficient rigidity to be driven into a tumor without deflection to provide for controlled and precise placement of the radioactive material. That is, the hollow needle will not flex during insertion into the tumor such that the needle will be driven into the tumor in a straight line.

The rigidity or non-deflectability of the delivery system can be controlled by selection of the polymer used and dimensions of the delivery system.

By making the interstitial delivery system substantially non-deflecting, the system is self-guidable and is preferably used without any assisting device such as hollow needles. The placement and removal of hollow needles in the cancerous tumor may transfer cancer cells to healthy tissue which is undesirable. The new self-guiding delivery system of the present invention does not suffer from this deficiency.

The advantages of the improved interstitial delivery systems of the present invention are:
1. The substantially non-deflecting members provide access into tight areas and can be placed in the most suitable direction.
2. Fixed linear positioning of seeds to minimize "hot" and "cold" spots.
3. The normal tissue is spaced away from the seed surface by the thickness of the body of polymer to decrease necrosis from a high local dose.
4. Improved handling and a labeling of the seeds will reduce exposure and error.
5. Increased speed of implant results in reduced surgical time and personal exposure.

The illustrated intracavitational radiation therapy device embodiments of the invention (Figs. 15-23) comprise a template of material which is absorbable in animal tissue in the form of a body of a geometric shape generally conforming to the cavity which remains after a tumor has been removed. A plurality of radioactive sources, such as seeds, are encapsulated in the center of the body.

The body can be made of any natural or synthetic absorbable materials. Example of natural absorbable materials are the polyester amides from glycolic or lactic acids such as the polymers and copolymers of glycolide and lactide, and polydioxanone.

The absorbable material should preferably maintain its integrity for from 1 to 14 days. Preferably the material should be absorbed in living tissue in a period of time of from about 70 to 120 days. It is preferred that as little absorbable material as possible be used in the delivery systems of the present invention.

The seeds can be of various types having low energy and low half-life such as iodine seeds, known as I-125 seeds, consisting of a welded titanium capsule containing iodine 125 absorbed on a silver rod. Iridium seeds, designated Ir-192 seeds, can also be used.

In the form shown in Figs. 15-17, the body 310 comprises a hollow exterior ovoid or sphere 311 defined by an exterior, thin wall which has a central portion 312 defining a diametral cavity 313 receiving the seeds 314. One end of the cavity is closed by a plastic plug or closure 315 of absorbable material; the other end is a closed tube end as shown.

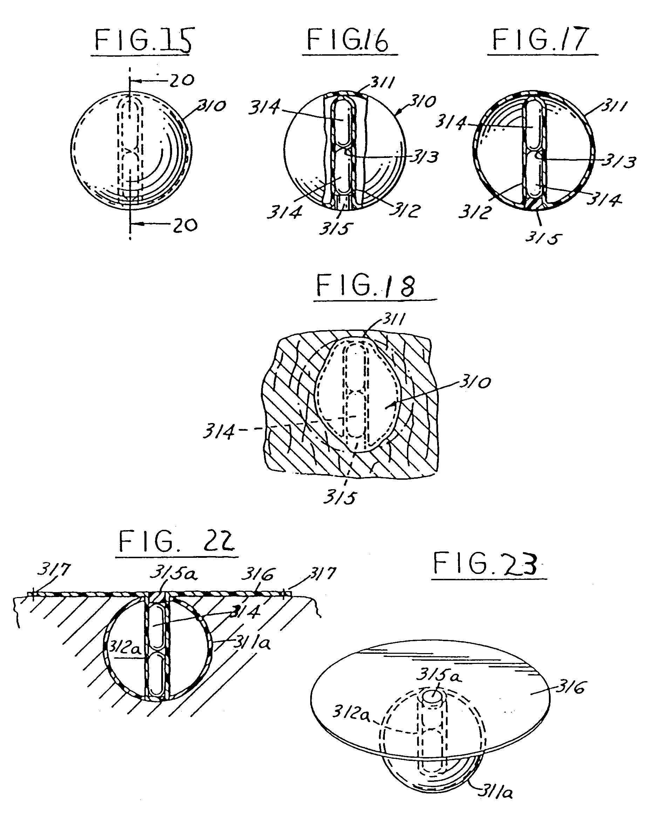

When a tumor is removed from a body such as the brain leaving a cavity, the body 310 with the seeds is placed in the cavity. The hollow defining thin wall 311 of the body has sufficient flexibility such that as the half life of the radioactive source is exceeded and the body 310 becomes absorbed, the tissue of the brain normally moves inwardly as shown in Fig. 18.

The provision of the body with the hollow sphere defined by thin wall 311 and the diametral portion 312 provides a light weight delivery system which also minimizes the amount of absorbable material left in the brain.

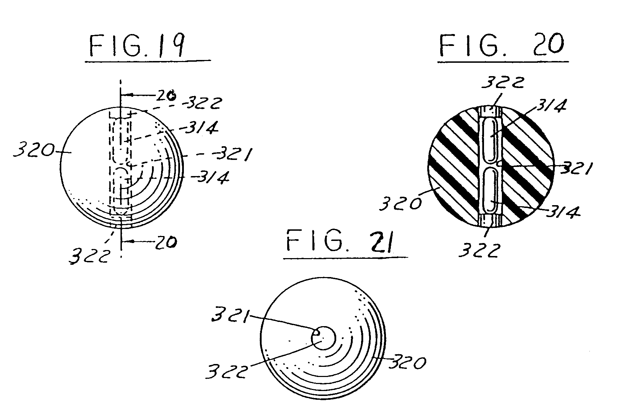

In the form shown in Figs. 19, 20 and 21, the body 320 is solid and includes a diametral cavity 321 receiving the seeds 314, both ends of the cavity being closed by plugs 322.

In a typical example:
1. A craniotomy is performed for surgical debulking of the tumor.
2. A properly shaped body is selected (spherical or cylindrical) and diameter (.5 to 3 cm) absorbable polymer ovoid for implant.
3. The radioactive sources, such as I-125 seeds, are inserted into the ovoid to deliver a dose of 3000 rads over one year (source life) at a distance of 2 cm from the ovoid surface.

The advantages of using absorbable material in implants includes the light weight and lack of tissue reaction. Light weight bodies are preferred and are achieved in the hollow form of Figs. 15-17. The importance of this in the brain is immediately seen in that the tissues would not be able to support a heavy object without migration due to gravity. A heavy object implanted in the brain may cause discomfort.

The fact that the body 310, 320 is absorbable has the advantage of eliminating itself as a nitious for bacterial contamination. This is of prime importance in treating brain tumors with its inability to tolerate infection.

The importance of decreasing the ratio of dose at the implant surface can be appreciated both theoretically and clinically. Dose rate decreases as the square of the distance. In malignant gliomas of the brain, 96% of the failures are due to local recurrence within two centimeters of the primary tumor. This is due to the local invasiveness of the primary tumor. After a debulking procedure, there will be a high concentration of microscopic disease around the surgical defect, presumed to be approximately 2 cm. This volume of tissue requires a boosting dose on the order of 6000 rads to 7000 rads as shown by previous experience in head and neck tumors as well as in brain tumors. Usually 4500 rads are given by large field and a boosting dose via the implant for an additional 3000 rads. Implanting a nude source of radioactive I-125 the dose will be 100,000 rads for a dose of 3000 rads at the surface. By implanting the source in a spherical or cylindrical container, the surface dose will be 5,000 rads for a 2 cm diameter ovoid. Thus, the ratio in surface dose by using the ovoid range from 6000 to 5000 should significantly reduce the hazard of radiation necrosis.

The form of device shown in Figs. 22 and 23 is similar to that shown in Figs. 15-18 with the addition of a sheet of non-absorbable plastic, such as nylon, fixed tangentially to the sphere 311 and the closure 315 provided so that it is in the central portion 312a which is tangential to the sheet 316.

In use, the sphere is placed within the cavity remaining after the tumor is removed and the sheet 316 attached thereto is utilized for suturing to the brain at 317.

In all the embodiments that are shown in Figs. 15-22, the geometric shape is a close or closed geometric shape such that a closed curve is produced when an imaginary plane extends through the shape. The closed curve is principally defined at the periphery of the shape.

## Claims

1. A delivery system for radiation therapy comprising an absorbable template (10, 146, 152, 160, 220, 230, 250, 310) made of a material absorbable in living tissue and radioactive seeds (15, 148, 170, 222, 234, 254, 314) supported by the template in a predetermined array, characterized in that each said radioactive seed is individually pre-encapsulated by the template such that said radioactive seeds of said array are fixed in stationary positions relative to each other upon implantation of said template in a patient.

2. A delivery system as claimed in Claim 1, wherein said template is a supporting structure for a two dimensional fixed array of radioactive seeds (10, 146, 152, 160).

3. A delivery system as claimed in Claim 2, wherein said template is a conformable sheet material (146, 152, 160) which is absorbable in living tissue, and said radioactive seeds (148, 170) are predeterminedly dispersed in said sheet material.

4. A delivery system as claimed in Claim 3, wherein said sheet material comprises a continuous film (146, 160) having the seeds dispersed in a predetermined pattern in the film.

5. A delivery system as claimed in Claim 4, wherein said radioactive seeds are provided in longitudinally spaced relation in said continuous film (146, 160).

6. A delivery system as claimed in Claim 3, wherein the sheet material comprises a pair of films (162, 164) secured together in spaced parallel rows and said radioactive seeds (170) are disposed between the films in the areas between said parallel rows, said films also being secured together between adjacent radioactive seeds in each row to separately encapsulate said seeds.

7. A delivery system as claimed in Claim 6, wherein the radioactive seeds (170) are longitudinally aligned.

8. A delivery system as claimed in Claim 1, wherein said template is an elongated, substantially non-deflecting member (220, 230, 250) made from a material which is absorbable in living tissue, said member having a length substantially greater than its width, and said radioactive seeds (222, 234, 254) are supported and encapsulated in said member in said predetermined and fixed array.

9. A delivery system as claimed in Claim 8, wherein said radioactive seeds (222, 234, 254) are provided in longitudinally spaced relation to said member.

10. A delivery system as claimed in Claim 8 or Claim 9, including a tapered end (224, 242, 252) on said body to facilitate insertion into a tumor.

11. A delivery system as claimed in any one of Claims 8-15, wherein said non-deflecting member (230) includes a plurality of interconnected segments (232) at least some of which have said radioactive seeds (234) encapsulated therein.

12. A delivery system as claimed in Claim 11, wherein said segments (232) are interconnected by an integral projection (236) on one end of each segment and a complementary recess (238) in the other end of said segment into which the projection of the adjacent segment extends and is frictionally held.

13. A delivery system as claimed in any one of Claims 8-10, wherein said non-deflecting member comprises a hollow tube (250) of absorbable material having said radioactive seeds (254) supported and encapsulated therein in said predetermined and fixed array by means of spacers (256) of absorbable material between said radioactive seeds.

14. A delivery system as claimed in any one of Claims 8-10, wherein said member (220) includes a plurality of narrow cross-sectional break regions (226) longitudinally disposed along the length of said member.

15. A delivery system as claimed in Claim 20, wherein said radioactive seeds (222) are interspersed among said break regions.

16. A delivery system as claimed in Claim 1, wherein said template comprises:
a body (310) of the material which is absorbable in animal tissue,
said body having a geometrical shape conforming generally with a cavity which is to be treated after removal of a tumor, and
a plurality of radioactive seeds (314) supported by and encapsulated within the body.

17. A delivery system as claimed in Claim 16, wherein said body (310) has a hollow exterior wall (311) and an internal chamber (312) containing said radioactive seeds.

18. A delivery system as claimed in Claim 17, wherein said body (310) is spherical.

19. A delivery system as claimed in any one of Claims 16-18, wherein said body (310) includes a removable closure (315) for providing loading of the seeds prior to use.

20. A delivery system as claimed in any one of Claims 16-19, including a sheet (316) of non-absorbable material attached to said body (310).

21. A delivery system as claimed in Claim 19 and Claim 20, wherein said removable closure (315) is generally in the plane of said sheet (316).

22. A delivery system as claimed in any preceding claim, wherein said material absorbable in living tissue is a synthetic polymer.

23. A delivery system as claimed in Claim 22, wherein said synthetic polymer is polydioxanone.

24. A delivery system as claimed in Claim 22, wherein said synthetic polymer is a copolymer of lactide and glycolide.

## Patentansprüche

1. Ein System zur Erteilung von Strahlungstherapie, umfassend eine absorbierbare Schablone (10, 146, 152, 160, 220, 230, 250, 310) aus einem in lebendem Gewebe absorbierbaren Stoff und durch die Schablone in einer vorbestimmten Anordnung abgestützte radioaktive Strahler (15, 148, 170, 222, 234, 254, 314), dadurch gekennzeichnet, daß jeder der besagten radioaktiven Strahler im voraus individuell innerhalb der Schablone gekapselt wird, so daß die besagten radioaktiven Strahler der besagten Anordnung bei Implantierung der besagten Schablone in einen Patienten in im Verhältnis zueinander feststehenden Lagen angebracht sind.

2. Ein Erteilungssystem nach Anspruch 1, bei dem die besagte Schablone eine Abstützstruktur für eine zweidimensionale feste Anordnung radioaktiver Strahler (10, 146, 152, 160) ist.

3. Ein Erteilungssystem nach Anspruch 2, bei dem die besagte Schablone ein entsprechendes, in lebendem Gewebe absorbierbares Plattenmaterial (146, 152, 160) ist und die besagten radioaktiven Strahler (148, 170) auf vorbestimmte Weise innerhalb des besagten Plattenmaterials dispergiert sind.

4. Ein Erteilungssystem nach Anspruch 3, bei dem das besagte Plattenmaterial einen kontinuierlichen Film (146, 160) umfaßt und die Strahler in einem vorbestimmten Muster innerhalb des Films dispergiert sind.

5. Ein Erteilungssystem nach Anspruch 4, bei dem die besagten radioaktiven Strahler in dem besagten kontinuierlichen Film der Länge nach in Abstand voneinander angeordnet sind.

6. Ein Erteilungssystem nach Anspruch 3, bei dem das Plattenmaterial zwei Filme (162, 164) umfaßt, die in voneinander in Abstand angeordneten parallelen Reihen miteinander verbunden sind, und die besagten radioaktiven Strahler (170) in den Bereichen zwischen den besagten parallelen Reihen zwischen den Filmen angeordnet sind, wobei die besagten Filme auch zwischen anschließenden radioaktiven Strahlern in jeder Reihe miteinander verbunden sind, so daß sie die besagten Strahler getrennt kapseln.

7. Ein Erteilungssystem nach Anspruch 6, bei dem die radioaktiven Strahler (170) der Länge nach miteinander ausgerichtet sind.

8. Ein Erteilungssystem nach Anspruch 1, bei dem die besagte Schablone ein längliches, im wesentlichen unbiegsames Element (220, 230, 250) aus einem in lebendem Gewebe absorbierbaren Stoff ist, wobei die Länge des besagten Elements erheblich größer ist als dessen Breite und die besagten radioaktiven Strahler (222, 234, 254) innerhalb des besagten Elements in der besagten vorbestimmten und festen Anordnung abgestützt und gekapselt sind.

9. Ein Erteilungssystem nach Anspruch 8, bei dem die besagten radioaktiven Strahler (222, 234, 254) im Verhältnis zu dem besagten Element der Länge nach und in Abstand voneinander vorgesehen sind.

10. Ein Erteilungssystem nach Anspruch 8 oder Anspruch 9, einschließlich eines sich verjüngenden Endes (224, 242, 252) an dem besagten Element, um dessen Einführung in einen Tumor zu erleichtern.

11. Ein Erteilungssystem nach einem der Ansprüche 8 bis 15, bei dem das besagte unbiegsame Element (230) eine Mehrzahl von miteinander verbundenen Segmenten (232) umfaßt, wobei innerhalb von mindestens einigen dieser Segmente besagte radioaktive Strahler (234) gekapselt sind.

12. Ein Erteilungssystem nach Anspruch 11, bei dem die besagten Segmente (232) miteinander durch einen integrierenden Vorsprung (236) an einem Ende jedes Segments und eine komplementäre Aussparung (238) im anderen Ende des besagten Segments verbunden sind, wobei sich der Vorsprung des anschließenden Segments in die besagte Aussparung erstreckt und darin durch Reibung festgehalten wird.

13. Ein Erteilungssystem nach einem der Ansprüche 8 bis 10, bei dem das besagte unbiegsame Element ein hohles Rohr (250) aus absorbierbarem Stoff umfaßt, wobei die besagten radioaktiven Strahler (254) in der besagten vorbestimmten und festen Anordnung durch Distanzstücke (256) aus absorbierbarem Stoff zwischen den besagten radioaktiven Strahlern abgestützt und gekapselt sind.

14. Ein Erteilungssystem nach einem der Ansprüche 8 bis 10, bei dem das besagte Element (220) eine Mehrzahl von schmalen, in Querrichtung verlaufenden Brechzonen (226) umfaßt, die entlang dem besagten Element der Länge nach vorgesehen sind.

15. Ein Erteilungssystem nach Anspruch 20, bei dem die besagten radioaktiven Strahler (222) zwischen den besagten Brechzonen eingefügt sind.

16. Ein Erteilungssystem nach Anspruch 1, bei dem die besagte Schablone folgende Teile umfaßt:
einen Körper (310) aus dem in tierischem Gewebe absorbierbaren Stoff,
wobei die geometrische Form des besagten Körpers im allgemeinen einem nach Entfernung eines Tumors zu behandelnden Hohlraum entspricht, und
eine Mehrzahl von radioaktiven Strahlern (314), die durch den besagten Körper abgestützt und innerhalb des besagten Körpers gekapselt sind.

17. Ein Erteilungssystem nach Anspruch 16, bei dem der besagte Körper (310) eine hohle Außenwand (311) und eine die besagten radioaktiven Strahler enthaltende Innenkammer (312) umfaßt.

18. Ein Erteilungssystem nach Anspruch 17, bei dem der besagte Körper (310) kugelförmig ist.

19. Ein Erteilungssystem nach einem der Ansprüche 16 bis 18, bei dem der besagte Körper (310) einen entfernbaren Verschluß (315) umfaßt, so daß die Strahler vor dem Gebrauch eingeführt werden können.

20. Ein Erteilungssystem nach einem der Ansprüche 16 bis 19, einschließlich einer Platte (316) aus nicht absorbierbarem Stoff, die an dem besagten Körper (310) angebracht ist.

21. Ein Erteilungssystem nach Anspruch 19 und Anspruch 20, bei dem der besagte entfernbare Verschluß (315) im allgemeinen in der Ebene der besagten Platte (316) liegt.

22. Ein Erteilungssystem nach einem der vorstehenden Ansprüche, bei dem der besagte in lebendem Gewebe absorbierbare Stoff ein synthetischer Polymer ist.

23. Ein Erteilungssystem nach Anspruch 22, bei dem der besagte synthetische Polymer Polydioxanon ist.

24. Ein Erteilungssystem nach Anspruch 22, bei dem der besagte synthetische Polymer ein Copolymer von Lactid und Glycolid ist.

## Revendications

1. Système de distribution pour radiothérapie comprenant une matrice absorbable (10, 146, 152, 160, 220, 230, 250, 310) constituée d'une matière absorbable dans les tissus vivants et de semences radioactives (15, 148, 170, 222, 234, 254, 314) supportées par la matrice dans un alignement prédéterminé, caractérisé en ce que chaque semence radioactive est pré-encapsulée individuellement par la matrice de telle sorte que lesdites semences radioactives dudit alignement sont fixées en position fixe les unes par rapport aux autres lors de l'implantation de ladite matrice dans un patient.

2. Système de distribution selon Revendication 1, dans lequel ladite matrice est une structure de support pour un alignement fixe bidimensionnel de semences radioactives (10, 146, 152, 160).

3. Système de distribution selon Revendication 2, dans lequel ladite matrice est une matière en feuille souple (146, 152, 160) qui est absorbable dans les tissus vivants, et où lesdites semences radioactives (148, 170) sont dispersées de façon prédéterminée dans ladite matière en feuille.

4. Système de distribution selon Revendication 3, dans lequel ladite matière en, feuille comprend une pellicule continue (146, 160) dans laquelle les semences sont dispersées suivant une disposition Prédéterminée.

5. Système de distribution selon Revendication 4, dans lequel lesdites semences radioactives sont fournies dans une relation d'espacement longitudinal dans ladite pellicule continue (146, 160).

6. Système de distribution selon Revendication 3, dans lequel la matière en feuille comprend une paire de pellicules (162, 164) fixées ensemble en rangées parallèles espacées et où lesdites semences radioactives (170) sont disposées entre les pellicules dans les zones comprises entre lesdites rangées parallèles, lesdites pellicules étant aussi fixées ensemble entre les semences radioactives contiguës dans chaque rangée pour encapsuler séparément lesdites semences.

7. Système de distribution selon Revendication 6, dans lequel les semences radioactives (170) sont alignées longitudinalement.

8. Système de distribution selon Revendication 1, dans lequel ladite matrice est un élément allongé, substantiellement inflexible (220, 230, 250) réalisé en matériau absorbable dans les tissus vivants, ledit élément ayant une longueur substantiellement plus grande que sa largeur, et où lesdites semences radioactives (222, 234, 254) sont supportées et encapsulées dans ledit élément dans ledit alignement prédéterminé et fixe.

9. Système de distribution selon Revendication 8, dans lequel lesdites semences radioactives (222, 234, 254) sont fournies dans une relation d'espacement longitudinal par rapport audit élément.

10. Système de distribution selon Revendication 8 ou Revendication 9, comprenant une extrémité conique (224, 242, 252) sur ledit corps pour faciliter l'insertion dans une tumeur.

11. Système de distribution selon l'une quelconque des Revendications 8-15, dans lequel ledit élément inflexible (230) comprend une pluralité de segments interconnectés (232) dans au moins certains desquels sont encapsulées lesdites semences radioactives (234).

12. Système de distribution selon revendication 11, dans lequel lesdits segments (232) sont interconnectés par une saillie intégrée (236) à une extrémité de chaque segment et un évidement complémentaire (238) dans l'autre extrémité dudit segment, dans lequel la saillie du segment contigu s'étend et est retenue par friction.

13. Système de distribution selon l'une quelconque des Revendications 8-10, dans lequel ledit élément inflexible comprend un tube creux (250) de matière absorbable dans lequel lesdites semences radioactives (254) sont supportées et encapsulées dans ledit alignement prédéterminé et fixe au moyen d'entretoises (256) de matière absorbable entre lesdites semences radioactives.

14. Système de distribution selon l'une quelconque des Revendications 8-10, dans lequel ledit élément (220) comprend une pluralité de régions de rupture de section étroite (226) disposées longitudinalement sur la longueur dudit élément.

15. Système de distribution selon Revendication 20, dans lequel lesdites semences radioactives (222) sont réparties parmi lesdites régions de rupture.

16. Système de distribution selon Revendication 1, dans lequel ladite matrice comprend:
un corps (310) en matière absorbable dans les tissus animaux,
ledit corps ayant une forme géométrique généralement conforme à la cavité qui doit être traitée après l'ablation d'une tumeur, et
une pluralité de semences radioactives (314) supportées par le corps et encapsulées dans celui-ci.

17. Système de distribution selon Revendication 16, dans lequel ledit corps (310) a une paroi extérieure creuse (311) et une chambre intérieure (312) contenant lesdites semences radioactives.

18. Système de distribution selon Revendication 17, dans lequel ledit corps (310) est sphérique.

19. Système de distribution selon l'une quelconque des Revendications 16-18, dans lequel ledit corps (310) comprend une fermeture amovible (315) pour permettre le chargement des semences avant utilisation.

20. Système de distribution selon l'une quelconque des Revendications 16-19, comprenant une feuille (316) de matière non absorbable fixée audit corps (310).

21. Système de distribution selon Revendication 19 et Revendication 20, dans lequel ladite fermeture amovible (315) est généralement dans le plan de ladite feuille (316).

22. Système de distribution selon l'une quelconque des revendications précédentes, dans lequel ledit matériau absorbable dans les tissus vivants est un polymère synthétique.

23. Système de distribution selon Revendication 22, dans lequel ledit polymère synthétique est du polydioxanone.

24. Système de distribution selon Revendication 22, dans lequel ledit polymère synthétique est un copolymère de lactide et de glycolide.
